# EUROPEAN PATENT APPLICATION

(11) **EP 2 047 886 A1**
(43) Date of publication of application: **15.04.2009**
(21) Application number: 07791359.8
(22) Date of filing: 26.07.2007
(51) Int. Cl.: A61N 1/32

(54) **LOW FREQUENCY THERAPY DEVICE AND ITS CONTROL METHOD**

(30) Priority: 01.08.2006 JP 2006210203
(71) Applicant: Omron Healthcare Co., Ltd., Kyoto 615-0084 (JP)
(72) Inventor: MIKI, Akitoshi, Kyoto-shi, Kyoto 6150084 (JP); ASAI, Rika, Kyoto-shi, Kyoto 6150084 (JP); MIZUTA, Yoshikazu, Kyoto-shi, Kyoto 6150084 (JP); KURASE, Koji, Osaka-shi, Osaka 5300005 (JP)
(74) Representative: Wilhelms · Kilian & Partner Patentanwälte
(86) International application number: PCT/JP2007/064659
(87) International publication number: WO 2008/015957

(57) **Abstract**

A low frequency therapy apparatus includes current generating means for generating a treatment current having an amplitude modulated waveform; a pad with a plurality of electrodes, which conduction is individually switchable, for outputting the treatment current through one or a plurality of electrodes in a conduction state; and control means for switching the conduction of the electrode during a period in which the amplitude becomes smaller than a predetermined threshold value while maintaining the waveform of the treatment current. The conduction of the electrode is thereby smoothly switched without giving an uncomfortable feeling to a user.

## Description

### TECHNICAL FIELD

The present invention relates to a switch control of an electrode of a low frequency therapy apparatus.

### BACKGROUND ART

A low frequency therapy apparatus for performing treatment by flowing low frequency current between a pair of pads attached to a body is known. In this type of low frequency therapy apparatus, a user may feel a shock when an electric stimulus is suddenly changed due to a change in a treatment mode and the like. Conventionally, the mode cannot be changed unless the user lowers an output level with a volume switch, or control is made to automatically lower the output level when switching the mode (see Patent Document 1).
[Patent Document 1] Japanese Unexamined Patent Publication No. 9-234252

### DISCLOSURE OF THE INVENTION

### PROBLEMS TO BE SOLVED BY THE INVENTION

The present inventors have reviewed a low frequency therapy apparatus including a plurality of electrodes, which conduction can be switched, and having a function of switching the conduction of each electrode while outputting a low frequency current for treatment. In the review, it was found that shock as in mode switching is felt even when switching conduction/disconnection of the electrode.

However, conventional countermeasures to manually or automatically lower an output level in mode switching cannot be applied to when switching the electrode. This is because the electrode is switched during the output of the low frequency current (i.e., during treatment), and thus the user may feel uncomfortable during treatment if the output level is lowered for every electrode switching.

In view of the above situations, it is an object of the present invention to provide a technique enabling the conduction of the electrode to be smoothly switched without giving an uncomfortable feeling to the user.

### MEANS FOR SOLVING THE PROBLEMS

The present invention adopts the following configuration to achieve the above object.

A low frequency therapy apparatus of the present invention includes current generating means for generating a treatment current having an amplitude modulated waveform; a plurality of electrodes, which conduction is individually switchable, for outputting the treatment current through one or a plurality of electrodes in a conduction state; and control means for switching the conduction of the electrode during a period in which the amplitude becomes smaller than a predetermined threshold value while maintaining the waveform of the treatment current.

A pad arranged with the plurality of electrodes is preferably arranged. This is so that attachment of a plurality of electrodes to the body is facilitated. The present invention is also applicable to a configuration in which each electrode is arranged on different pads.

The treatment current having the amplitude modulated waveform has a period in which the amplitude (output level) periodically becomes small. The present invention uses such period to switch the conduction of the electrode when the amplitude becomes smaller than the threshold value. The shock in time of switching the electrode thus becomes as small as possible. Furthermore, the user does not feel an uncomfortable feeling in treatment since the waveform of the treatment current is maintained.

The control means preferably has an automatic switching function of automatically switching the conduction of the electrode according to a program. In this case, the period in which the amplitude becomes smaller than the predetermined threshold value and a timing of switching the conduction of the electrode are preferably synchronized in the program.

The control means preferably has a manual switching function of switching the conduction of the electrode according to a switch instruction input by a user. In this case, the control means waits until the period in which the amplitude becomes smaller than the predetermined threshold value to switch the conduction of the electrode when the switch instruction is input.

The present invention may relate to a low frequency therapy apparatus including at least one part of the above described means, the present invention may relate to a method of controlling the low frequency therapy apparatus including at least one part of the process, or a program for realizing such method and a recording medium recorded with the program. Each of the above described means and processes can be combined with each other as much as possible to configure the present invention.

For instance, in the method of controlling the low frequency therapy apparatus according to the present invention, the low frequency therapy apparatus including a plurality of electrodes, which conduction is switchable, generates the treatment current having an amplitude modulated waveform, outputs the treatment electrodes through one or a plurality of currents in a conduction state; and switches the conduction of the electrode during a period in which the amplitude becomes smaller than a predetermined threshold value while maintaining the waveform of the treatment current.

According to the present invention, the conduction of the electrode can be smoothly switched without giving an uncomfortable feeling to the user.

### BRIEF DESCRIPTION OF THE DRAWINGS

Fig. 1 is a view showing an outer appearance of a low frequency therapy apparatus.
Fig. 2 is a block diagram schematically showing a hardware configuration of the low frequency therapy apparatus.
Fig. 3 is a view showing one example of a treatment waveform using an alternate current.
Fig. 4 is a view showing an example of changing the number (an area) of the electrode.
Fig. 5 is a view showing an example of changing the position of the electrode.
Fig. 6 is a flowchart showing a flow of process in manual switching.

### DESCRIPTION OF REFERENCE SYMBOLS

- 100: Low frequency therapy apparatus
- 200: Therapy apparatus body
- 201: Operation unit
- 202: Display unit
- 203: Control unit
- 204: Current generating unit
- 205: Electrode switching unit
- 300: Pad
- 301: Electrode
- 302: Snap
- 400: Cord
- 401: Plug
- 402: Snap

### BEST MODE FOR CARRYING OUT THE INVENTION

Preferred embodiments of the present invention will be specifically described in an illustrative manner with reference to the drawings.

### <Outer appearance of low frequency therapy apparatus>

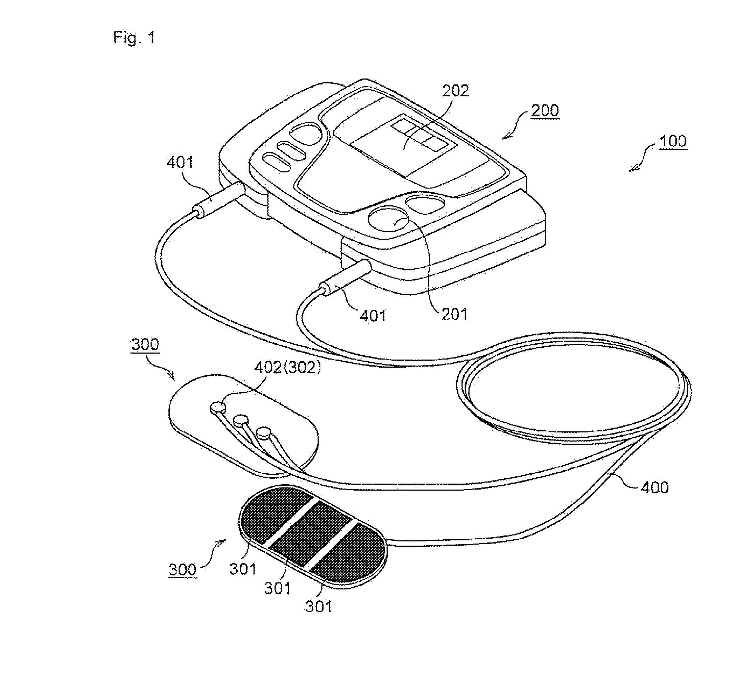

A low frequency therapy apparatus according to an embodiment of the present invention will be described with reference to Fig. 1. Fig. 1 is a view showing an outer appearance of a low frequency therapy apparatus.

A low frequency therapy apparatus 100 is schematically configured by a therapy apparatus body 200, a pair of pads 300 to be attached to a treatment site, and a cord 400 for electrically connecting the therapy apparatus body 200 and the pad 300.

The therapy apparatus body 200 is arranged with an operation unit 201 configured by a button, a volume switch and the like, and a display unit 202 configured by a liquid crystal display and the like. A user operates the operation unit 201 to switch a power ON/OFF, select a treatment mode, adjust strength and speed, switch electrodes, and perform other various settings.

The pad 300 is configured by a thin and highly flexible member. One surface (surface that contacts the body) of the pad 300 is formed with a plurality of (three) electrodes 301. Surfaces of such electrodes 301 are covered by a conductive gel material having adherence, and the electrodes are not particularly electrically insulated in between by way of the gel material.

The other surface of the pad 300 is arranged with a snap 302 corresponding to each electrode 301. A snap 402 of the cord 400 is fastened to the snap 302 on the pad 300 side, and a plug 401 of the cord 400 is inserted to the therapy apparatus body 200 to achieve connection between the therapy apparatus body 200 and the pad 300 (electrode 301).

### <Internal configuration of low frequency therapy apparatus>

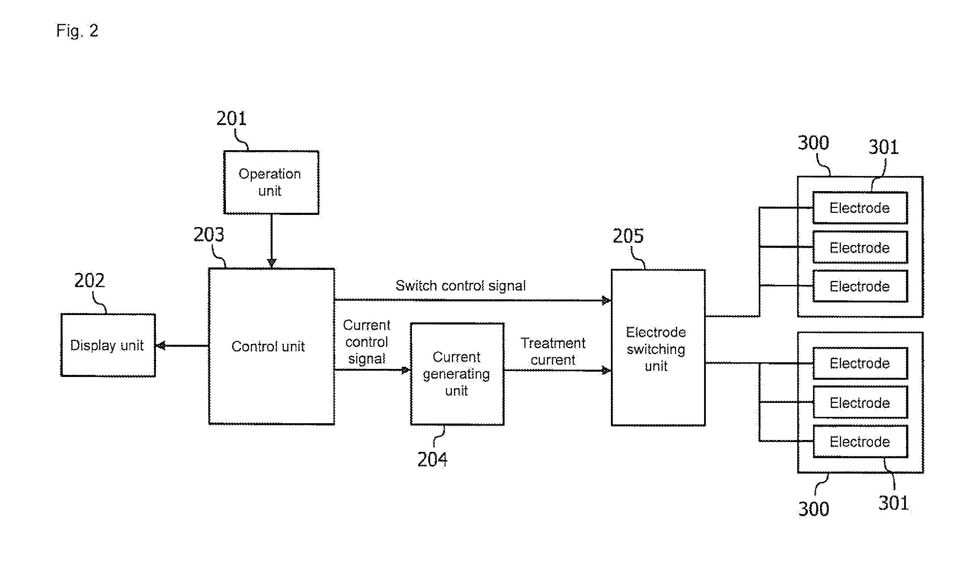

Fig. 2 is a block diagram schematically showing a hardware configuration of the low frequency therapy apparatus. As shown in Fig. 2, a control unit 203, a current generating unit 204, an electrode switching unit 205, a power supply (not shown), and the like are arranged inside the therapy apparatus body 200.

The control unit 203 is a circuit configured by a microcomputer. The signals from the operation unit 201 are input to the control unit 203. The control unit 203 has a function of controlling the display unit 202, the current generating unit 204, the electrode switching unit 205, and the like, or executing various calculation processes based on such inputs.

The current generating unit 204 is a circuit for generating a low frequency current (treatment current) to output from the pad 300. An alternative current having an amplitude modulated waveform is used for the low frequency current. The waveform and the amplitude of the modulation wave of the low frequency current are determined by a current control signal input from the control unit 203.

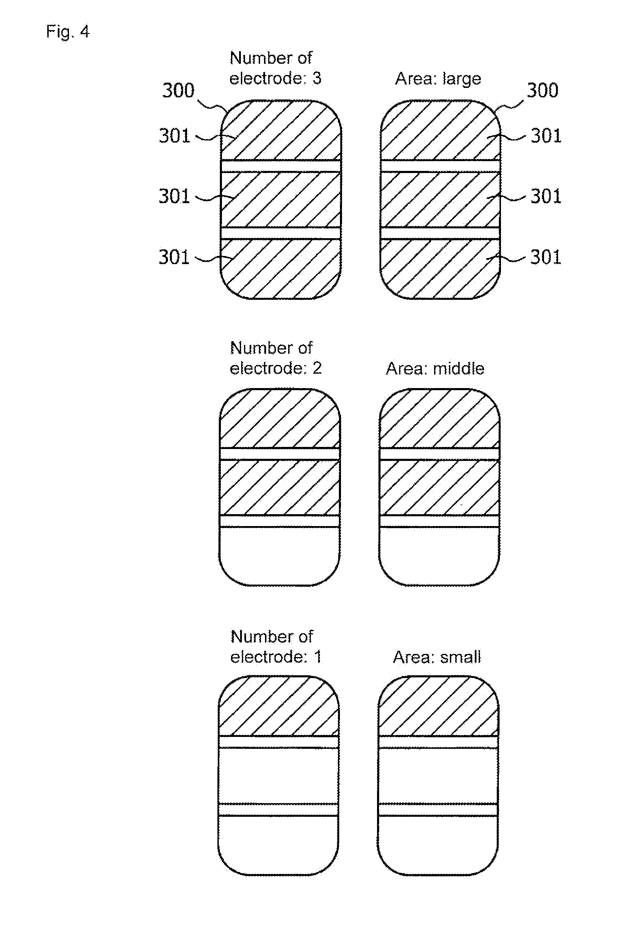

Fig. 4 shows an amplitude modulation wave in which a carrier wave frequency is about 4 kHz, a modulation wave frequency is about 122 Hz, and the maximum amplitude is about 35 V as one example of the treatment waveform using a treatment current. "Strength" is adjusted by changing the amplitude, and "speed" is adjusted by changing the modulation wave frequency.

The electrode switching unit 205 is a circuit having a function of switching conduction/disconnection of each six electrodes 301, and distributing the low frequency current to the electrodes 301 in a conduction state. The switching of the electrode 301 is controlled by a switch control signal input from the control unit 203.

### <Operation at a time of treatment>

An operation method and a therapy operation of the low frequency therapy apparatus 100 will now be described.

The user operates the operation unit 201 to select the treatment mode. For the treatment mode, the treatment site such as "shoulder", "arm", "lower back", and the like can be specified. An "automatic" mode in which the treatment method is programmed in advance may be selected. The user can also operate the volume switch to adjust the "strength" and the "speed".

The control unit 203 generates a current control signal according to the specified treatment mode, the strength, and the speed, and sends the signal to the current generating unit 204. The current generating unit 204 performs modulation and amplification based on the current control signal, and generates the low frequency current for treatment. The current of one system is distributed to each electrode 301 in the electrode switching unit 205, and is output to the body of the user through the plurality of electrodes 301. Muscles are electrically stimulated by the treatment current flowing between the pads and repeat contraction and relaxation, whereby a therapeutic effect same as massage is obtained.

### <Switch control of electrode>

In the low frequency therapy apparatus 100 of the present embodiment, a plurality of electrodes 301, which conduction is switchable, is arranged on each pad 300, and the conduction/disconnection of the electrode 301 can be manually or automatically switched during the output of the low frequency current. The low frequency current is distributed to one or the plurality of electrodes 301 in the conduction state, and output to the body through the electrodes 301 in the conduction state.

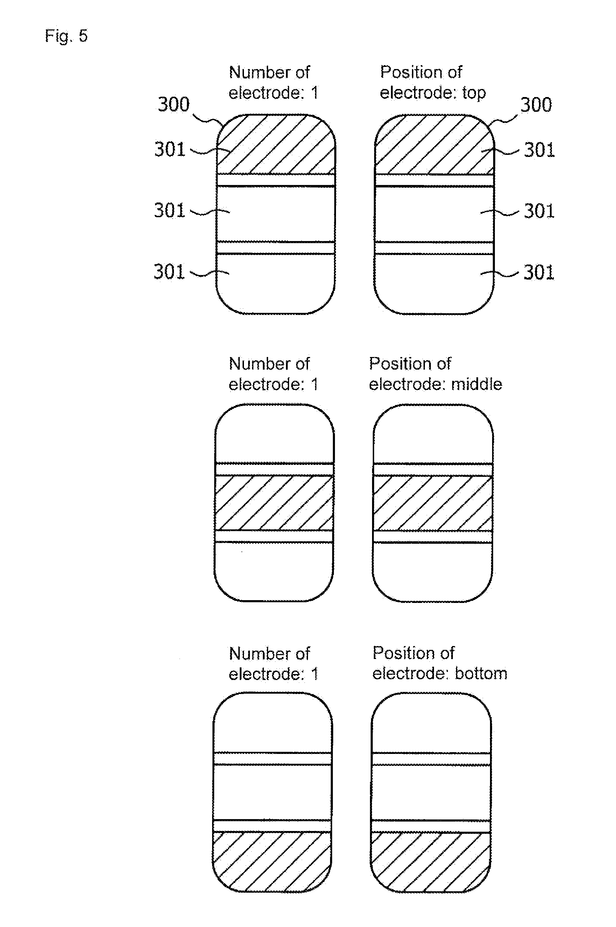

A switching example of the electrode is shown in Figs. 4 and 5. In such figures, a hatching portion represents the electrode in the conduction state. Fig. 4 is an example in which the number (an area) of the electrode is changed. If all of three electrodes 301 are conducted, the current is output from the entire pad, and thus the treatment site can be covered in a wide range. If only one or two electrodes are conducted, a pinpoint treatment can be performed. Fig. 5 is an example in which the position of the electrode is changed. The treatment site can be changed by changing the position of the electrode to conduct. Thus, the range of usage becomes wider and a flexible treatment becomes possible since the plurality of electrodes are arranged on one pad.

If the electrode is suddenly switched while outputting the low frequency current, the user may feel a shock. However, the user may feel uncomfortable during treatment if the output level is lowered for every electrode switching, as described above, and thus is not desirable.

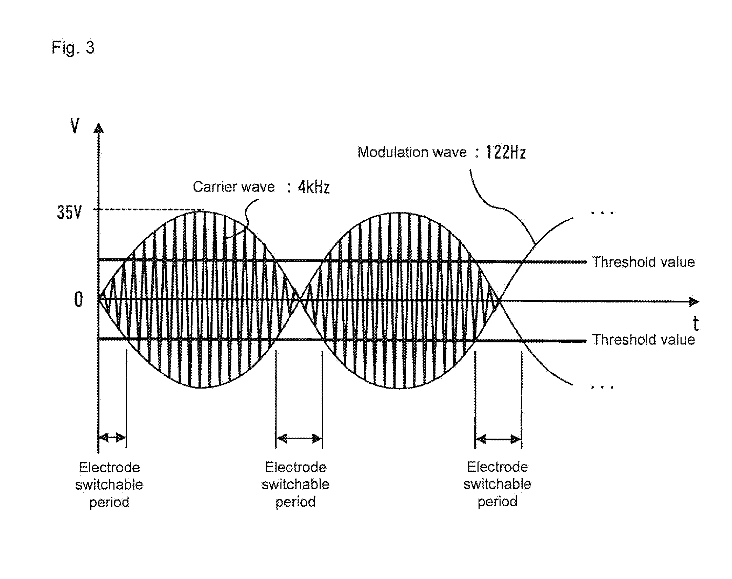

The low frequency therapy apparatus 100 of the present embodiment uses characteristics of the waveform of the treatment current. That is, as shown in Fig. 3, the treatment current output by the low frequency therapy apparatus 100 is an alternating current having an amplitude modulated waveform, and thus a period in which the amplitude (output level) periodically becomes small appears. Using this period, the conduction/disconnection of the electrode is switched when the amplitude of the modulation wave becomes smaller than a predetermined threshold value ("electrode switchable period" of Fig. 3).

The threshold value is set to a value of an extent the user cannot sense at all (or barely sense) the shock in time of electrode switching. However, since body feeling varies depending on the waveform and the frequency of the low frequency current, a size and a shape of the electrode, and the like, a specific numerical value is preferably determined by conducting clinical experiments for every model. Furthermore, since the body feeling also varies among individuals, a configuration in which the user can change the threshold value is also preferable. In the present embodiment, the threshold value is set to a value of 20% of a maximum amplitude. That is, the threshold value dynamically changes according to the set value of the "strength" by the volume switch. The threshold value is not set to a fixed value because the body feeling changes according to the strength of the treatment current, for example, a shock is felt when the electrode is switched at a timing of 10 V if the maximum amplitude of the treatment current is 10 V, whereas the shock is not felt even when the electrode is switched at the timing of 10 V if the maximum amplitude is 50 V.

### <Automatic switching>

When "automatic" is selected for the treatment mode, the control unit 203 automatically switches the conduction of each electrode 301 according to a program. In the program, the period in which the amplitude of the low frequency current becomes smaller than the threshold value and the timing of switching the conduction of the electrode are synchronized.

For instance, in the "automatic" mode of the treatment site "shoulder", an operation of first conducting three electrodes to perform treatment on the entire shoulder, and conducting only one electrode and performing the treatment while changing the relevant position in order is programmed. In this case, the number of electrodes is changed from "3" → "1" when the amplitude of the waveform of the treatment current becomes smaller than the threshold value. The change in the electrode position is also carried out when the amplitude of the waveform of the treatment current becomes smaller than the threshold value.

### (Manual switching)

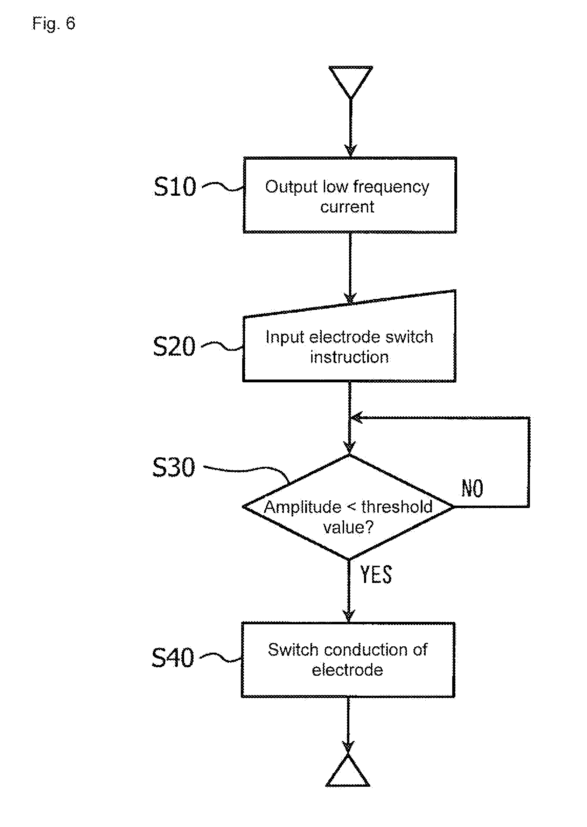

The user can switch the number and the position of the electrode to conduct by operating the operation unit 201. The flow of process in this case will be described along the flowchart of Fig. 6.

During the output of the low frequency current (step S10), if an instruction to switch the electrode is input by the user (step S20), the control unit 203 waits until the period in which the amplitude of the low frequency current becomes smaller than the threshold value (step S30). Since generating a current control signal for determining the amplitude of the low frequency current is also a function of the control unit 203, the waiting process can be realized in the internal processing of the control unit 203. When the amplitude becomes smaller than the threshold value, the control unit 203 sends a switch control signal to the electrode switching unit 205, and controls conduction/disconnection of the electrode 301 (step S40).

According to the embodiment described above, the shock in time of electrode switching becomes zero or as small as possible since the conduction of the electrode is switched when the amplitude of the low frequency current is small. Furthermore, the user does not feel uncomfortable during treatment since the treatment waveform of the low frequency current is maintained. A smooth switching of the electrode is thereby achieved.

It should be noted that the embodiment described above merely illustrates one specific example of the present invention. The scope of the present invention is not limited to the above embodiment, and various modifications may be made within the scope of the technical idea.

For instance, the number of electrodes of the pad is not limited to three. It may be two, or it may be three or more. The shape and a dividing manner of the electrode can also be appropriately changed. Furthermore, each electrode may be arranged on different pads instead of arranging a plurality of electrodes on one pad.

## Claims

1. A low frequency therapy apparatus comprising:
current generating means for generating a treatment current having an amplitude modulated waveform;
a plurality of electrodes, which conduction is individually switchable, for outputting the treatment current through one or a plurality of electrodes in a conduction state; and
control means for switching the conduction of the electrode during a period in which the amplitude becomes smaller than a predetermined threshold value while maintaining the waveform of the treatment current.

2. The low frequency therapy apparatus according to claim 1, further comprising a pad arranged with the plurality of electrodes.

3. The low frequency therapy apparatus according to claim 1 or 2, wherein
the control means has an automatic switching function of automatically switching the conduction of the electrode according to a program; and
the period in which the amplitude becomes smaller than the predetermined threshold value and a timing of switching the conduction of the electrode are synchronized in the program.

4. The low frequency therapy apparatus according to any one of claims 1 to 3, wherein
the control means has a manual switching function of switching the conduction of the electrode according to a switch instruction input by a user; and
the control means waits until the period in which the amplitude becomes smaller than the predetermined threshold value to switch the conduction of the electrode when the switch instruction is input.

5. A method of controlling a low frequency therapy apparatus, wherein the low frequency therapy apparatus including a plurality of electrodes, which conduction is switchable,
generates a treatment current having an amplitude modulated waveform;
outputs the treatment electrodes through one or a plurality of currents in a conduction state; and
switches the conduction of the electrode during a period in which the amplitude becomes smaller than a predetermined threshold value while maintaining the waveform of the treatment current.
